# EUROPÄISCHE PATENTSCHRIFT

(11) **EP 1 516 142 B1**
(45) Veröffentlichungstag und Bekanntmachung des Hinweises auf die Patenterteilung: **22.08.2007**
(21) Anmeldenummer: 03760582.1
(22) Anmeldetag: 16.04.2003
(51) Int. Cl.: F16L 37/084, F16L 37/10

(54) **VORRICHTUNG ZUM VERBINDEN VON EINER AUS FLEXIBLEM MATERIAL BESTEHENDEN KANÜLE MIT EINEM ROHR**
DEVICE FOR CONNECTION OF A CANNULA MADE FROM A FLEXIBLE MATERIAL TO A TUBE
DISPOSITIF POUR RELIER UNE CANULE EN MATERIAU SOUPLE A UN TUBE

(30) Priorität: 24.06.2002 DE 10228918; 24.06.2002 DE 20209987 U
(43) Veröffentlichungstag der Anmeldung: 23.03.2005
(73) Patentinhaber: Berlin Heart GmbH, 12247 Berlin (DE)
(72) Erfinder: OTT, Friedrich, 10711 Berlin (DE)
(74) Vertreter: Pfenning, Meinig & Partner GbR
(86) Internationale Anmeldenummer: PCT/EP2003/003968
(87) Internationale Veröffentlichungsnummer: WO 2004/001272

(56) Entgegenhaltungen:
- EP-A- 1 219 884
- DE-A- 3 715 911
- DE-A- 19 622 099
- DE-C- 4 129 397
- DE-U- 20 209 987

## Beschreibung

Die Erfindung bezieht sich auf eine Vorrichtung zum Verbinden von einer aus flexiblen Material bestehenden Kanüle (bzw. Schlauch) mit einem Rohr gemäß dem Oberbegriff des Anspruches 1.

Vorrichtungen zum Verbinden von Kanülen und Rohren sind bekannt, beispielsweise aus DE 4 129 397. So werden häufig Überwurfmuttern benutzt, um eine Verbindung zwischen Rohren bzw. Kanülen zu erreichen. Hierbei wird die Überwurfmutter mit einer Spannzange oder Olive (Stoßverbindung) kombiniert, um Rohre bzw. Kanülen miteinander zu verbinden. Beim Anziehen der Mutter wird eine Radialkraft auf die Kanüle übertragen. Mit der Überwurfmutter wird eine axiale Sicherung und eine radiale Dichtung erreicht. Diese Verbindungstechnik wird zur Zeit insbesondere dafür benutzt, um implantierbare Blutpumpen, bei denen Verbindungen zwischen einem in der Regel metallischem Rohr mit einer aus flexiblen Material bestehenden Kanüle erforderlich sind, zu verbinden.

Diese Art der bekannten Verbindungen zwischen Blutpumpe und Kanülen und Rohr hat die folgenden Nachteile:
- Ein Gewinde erfordert größere Wandstärken. Dadurch ergibt sich ein größerer Durchmesser an der Verbindung. Des Weiteren ist eine Gewichtzunahme durch mehr Material unumgänglich.
- Es sind keine reproduzierbaren und über einen langen Zeitraum konstanten Anschlußbedingungen gegeben. Ein definiertes Anzugsmoment ist nur bedingt möglich. Die dafür notwendige Verwendung eines Drehmomentenschlüssels hätte den Nachteil, dass der Chirurg ein weiteres Werkzeug zur Implantation und Explantation braucht. Durch ein undefiniertes Anzugsmoment können zum einem bei zu festem Anziehen Materialschädigungen entstehen (Silikon fließt bei hohen Kräften und Polyestergewebe sowie Dacron oder PTFE kann reißen) oder zum anderen sitzt die Mutter zu locker.
- Beim Fehlen einer Verdrehsicherung kann sich die Überwurfmutter durch Vibrationen lösen. Dies würde zwingend zu einer lebensbedrohlichen Leckage führen, da die Mutter die axiale Sicherheit und radiale Dichtung nicht mehr aufrecht erhalten kann.
- Die Überwurfmutter besitzt meistens ein Feingewinde. Dadurch ergibt sich eine lange Einschraublänge. Das bedeutet schwierige Handhabbarkeit und damit einen hohen Zeitaufwand für den Chirurgen.
- Die Überwurfmutter-Technik besitzt undefinierte und blutschädigende Übergänge. Dadurch besteht Hämolyse- und Thrombengefahr.
- Überwurfmutter-Technik ist immer auf ein bestimmtes Kanülenmaterial (Titan, Polyestergewebe-PTFE, Dacron, Silikon oder Polyurethan) angepaßt. Andere Kanülen-Materialien bedingen andere Konstruktionen.
- Bei den zur Zeit bekannten Systemen kann die Längenanpassung der Auslasskanüle auf die Patientenanatomie nur am distalen Ende (Aortenseite) erfolgen. Die freie Gestaltungsmöglichkeit des Kopfes der Auslasskanülen ist somit nicht mehr möglich. Dadurch kann sich beispielsweise bei den hauptsächlich benutzten Auslasskanülen aus Polyestergewebe ein undefinierter und blutschädigender Übergang ergeben. Des Weiteren können diese Auslasskanülen einknicken (kinking) und zu einer Flußminderung beim Fördern des Blutes führen.

Der Erfindung liegt die Aufgabe zugrunde, eine Vorrichtung zum Verbinden von aus flexiblen Materialien bestehenden Kanülen mit Rohren anzubieten, mit denen unkomplizierte, schnelle, sichere und reproduzierbare Bedingungen zum Verbinden und Entkoppeln von Kanüle und Rohr gegeben sind, insbesondere unter den Bedingungen eines operativen Eingriffes beim Anschluß einer herzunterstützenden oder herzersetzenden Blutpumpe.

Die Lösung der Aufgabe erfolgt mit den Merkmalen des Anspruches 1.

So ist die erfindungsgemäße Vorrichtung zum Verbinden von einer aus flexiblen Material bestehenden Kanüle mit einem Rohr, wobei ein am Rohr angesetzter Rohrstutzen z.B. eine Olive in die Kanüle eingeschoben ist, dadurch gekennzeichnet, dass ein auf einem Kanülenende bis zu einem Anschlag verschiebbar und drehbar angeordneter Klauenring mit einem auf einem Rohrende befestigten Rastring durch axiales Verschieben und Einrasten am Rastring verbindbar ist.

Auf dem Kanülenende ist ein Spannring angeordnet, der mit der Kanüle fest verbunden ist. Auf diesem Spannring ist der Klauenring verschiebbar und drehbar angeordnet. Vorteilhafte Weiterbildungen sind in den Unteransprüchen angegeben. Die Formgebung und die Materialwahl des Spannrings ermöglichen es vorteilhafterweise, die radiale Dichtung zu unterstützen, indem zusätzliche radiale Dichtkräfte erzeugt werden.

So ist es möglich, den Spannring aus elastischem Material, wie z.B. Silikon, oder auch aus unflexiblem Material, wie z.B. Metall, zu fertigen. Insbesondere lassen sich Spannringe aus Metall auch als geschlitzte Hülse ausführen.

In einer weiteren Ausgestaltung der Erfindung ist der Anschlag als endständiger Bund am Kanülenende ausgebildet, so dass beim Einrasten der Klauenring den endständigen Bund gegen den Rastring drückt und damit eine dichte Verbindung herstellt.

In einer weiteren Ausgestaltung der Erfindung ist vorgesehen, zwischen Bund und Klauenring einen Distanzring anzuordnen, der beim Einrastvorgang gegen den Bund drückt. Wenn der Bund aus flexiblem Material gefertigt ist, wird mittels des Distanzringes eine bessere Druckverteilung erreicht. Der Distanzring kann sowohl verschiebbar und drehbar als auch fest mit dem Klauenring verbunden, eingebaut sein.

In einer weiteren Ausgestaltung der Erfindung befindet sich auf dem Kanülenende ein Verstärkungsformteil, das mit der Kanüle fest verbunden ist und auf dem der Klauenring verschiebbar und drehbar angeordnet ist. Vorteilhafterweise kombiniert das Verstärkungsformteil den Spannring mit dem Bund.

In einer weiteren Ausgestaltung der Erfindung ist zwischen Spannring und Bund eine Nut vorgesehen, die den Distanzring aufnimmt. Der Distanzring kann unterschiedliche Formen aufweisen, die sich insbesondere aus den konstruktiven Erfordernissen ergeben können. Er wird unter Verformung des flexiblen Bundes in die Nut eingedrückt. Da beim Einrastvorgang des Klauenringes dieser gegen den Distanzring und der wiederum gegen den Bund drückt, entsteht so eine dichte Verbindung mit dem Rastring, so dass die radiale Dichtung durch die elastisch aufgeweitete Kanüle und den Spannring, in diesem Fall durch die axiale Dichtung mit Hilfe der Dichtflächen am Bund, ergänzt wird.

Es ist auch möglich, nur an der Stirnfläche des Bundes axial zu dichten. Der in die Kanüle ragende Rohrstutzen, z.B. als Olive ausgeführt, kann dann extrem kurz sein oder ganz entfallen, so dass die Gesamtlänge des Herzunterstützungssystems vorteilhafterweise verringert werden kann.

In einer weiteren vorteilhaften Ausgestaltung der Erfindung weist der Klauenring einen Grundring auf, an dem Klauen, die Klauenhalteflächen aufweisen, über Federgelenke am Grundring angeordnet sind. Der Grundring kann auch mit dem Distanzring fest verbunden sein.

In einer Weiterbildung der Erfindung weist der Rastring Halteflächen auf, an die die Klauenhalteflächen des Klauenringes anliegen.

In einer weiteren Ausgestaltung der Erfindung besteht das Verstärkungsformteil aus flexiblem Material, vorteilhafterweise aus dem gleichen Material, aus dem auch die Kanüle gefertigt ist, vorzugsweise Silikon.

In einer weiteren Ausgestaltung ist das Verstärkungsformteil integraler Bestandteil des Kanülenendes, das beispielsweise im Spritzgußverfahren hergestellt wird, so dass Kanüle und Verstärkungsformteil aus dem gleichen Material bestehen.

In einer weiteren Ausgestaltung weist der Rastring Steigflächen auf, die bei Drehung des Klauenrings relativ zum Rastring zu einem Aufspreizen der Klauen führen, so dass der Klauenring vom Rastring abgezogen werden kann, nachdem die Klauen die Rastringsteigflächen verlassen haben. Die Verbindung wird entriegelt.

In einer weiteren Ausgestaltung weist der Rastring eine Drehbegrenzung auf, an der bei Drehung des Klauenrings relativ zum Rastring eine nichtaufgespreizte Klaue anschlägt und so verhindert, dass der Klauenring über die Entriegelungsposition hinaus verdreht werden kann. Ein weiterer gleichartiger Anschlag kann spiegelsymmetrisch angeordnet sein, so dass unabhängig von der Drehrichtung des Klauenrings die Drehung in der Entriegelungsposition begrenzt wird.

In einer weiteren vorteilhaften Ausgestaltung der Erfindung weist die Kanüle zwei oder mehr hintereinander angeordnete Verstärkungsformteile auf. Das hat den Vorteil, dass beim Implantationsprozess der Pumpe die Kanüle kürzbar ist, ohne dass ein weiteres Verstärkungsformteil auf das Kanülenende aufgeschoben werden muss.

Die Erfindung hat die folgenden Vorteile:
- Es ist möglich, eine einfache, schnellere und sichere Verbindung einer flexiblen Kanüle mit einem Metallrohr unter den Bedingungen einer Implantation einer Blutpumpe vorzunehmen, wobei diese Verbindung durch Drehung des Klauenringes und axiales Abziehen des Klauenringes und der Kanüle wieder lösbar ist.
- Es liegt nur eine geringere Vergrößerung des Außendurchmessers an der Verbindungsstelle zwischen Kanüle und Rohr gegenüber dem Außendurchmesser der Kanüle bzw. des Rohres vor, die mit einem geringeren Gewicht verbunden ist.
- Es liegen reproduzierbare Anschlußbedingungen vor.
- Es erfolgt eine Trennung der axialen Sicherung und der radialen und/oder axialen Dichtung im Vergleich zu einer Überwurfmutterverbindung.
   Die axiale Sicherung gegen Trennung der Verbindung wird hierbei durch den Klauenring gewährleistet. Die radiale bzw. axiale Dichtung wird durch optimierte Durchmesserverhältnisse bzw. Dickenverhältnisse am Rohrstutzen (Olive) und an der Kanüle erreicht. Dadurch wird keine undefinierte axiale oder radiale Kraft auf die Kanüle aufgebracht, d.h. es erfolgt keine zusätzliche negative Beeinflussung des Kanülen-Materials. Des Weiteren entfällt eine zusätzliche Verdrehsicherung, da zum Lösen der Verbindung der Klauenring verdreht werden müsste, wozu ein Drehmoment erforderlich wäre, das ein Aufspreizen der Klauen durch Gleiten auf den Steigflächen des Rastringes bewirken könnte. Dieses Drehmoment würde nur, wenn es erforderlich ist, der Operateur aufbringen. Es liegt somit Selbstsicherung vor.
- Die erfindungsgemäße Schnappverbindung macht es möglich, einen idealen und dadurch blutschonenden Übergang von Kanüle zum Rohr zu erreichen. Die Verbindung zwischen dem als Olive ausgebildeten Rohrstutzen, in der Regel aus Titan bestehend, und der Kanüle weist praktisch keine Strömungshindernisse auf.
- Die erfindungsgemäße Vorrichtung funktioniert bei jedem bekannten Kanülenmaterial, ohne dass größere konstruktive Änderungen erforderlich wären.
- Für das Verbinden und Entkoppeln der erfindungsgemäßen Vorrichtung wird kein Spezialwerkzeug benötigt.
- Die Längenanpassung der Auslasskanüle erfolgt am pumpenseitigen Ende durch Abschneiden der nicht benötigten Verstärkungsformstücke. Der Klauenring wird dann wieder auf die Kanüle geschoben und das Distanzstück in die dem Kanülenende am nächsten liegende Nut eingeknüpft. Dadurch ist eine freie Gestaltungsmöglichkeit des Auslasskanülenendes möglich.

Die Erfindung wird anhand eines Ausführungsbeispieles und Zeichnungen näher erläutert.

Es zeigen
- Fig. 1a: eine schematische, axiale Schnittdarstellung eines Kanülenendes vor dem Zusammenfügen,
- Fig. 1b: eine schematische, axiale Schnittdarstellung des Rohrendes vor dem Zusammenfügen,
- Fig. 1c: eine schematische, axiale Schnittdarstellung der erfindungsgemäßen Zusammenfügung des Kanülenendes mit dem Rohrende,
- Fig. 2: einen Klauenring in perspektivischer Darstellung,
- Fig. 3 a: eine axiale Schnittdarstellung eines Kanülenendes mit Distanzring,

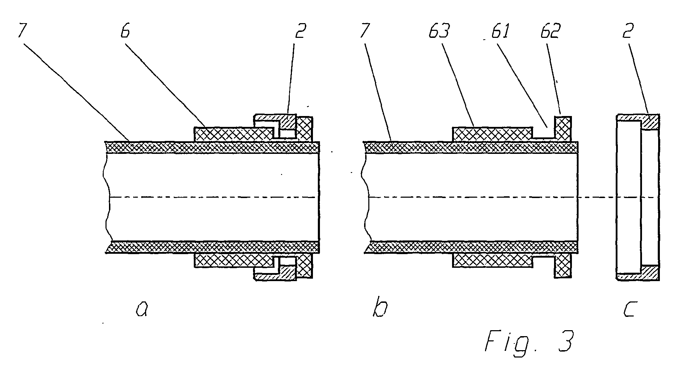

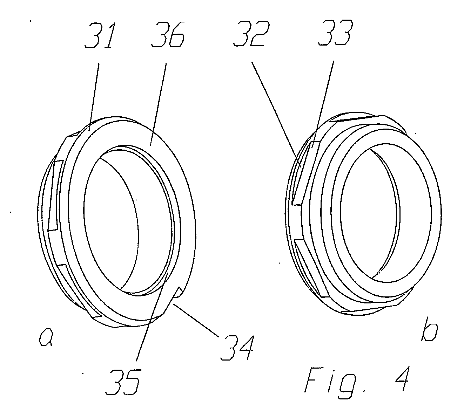

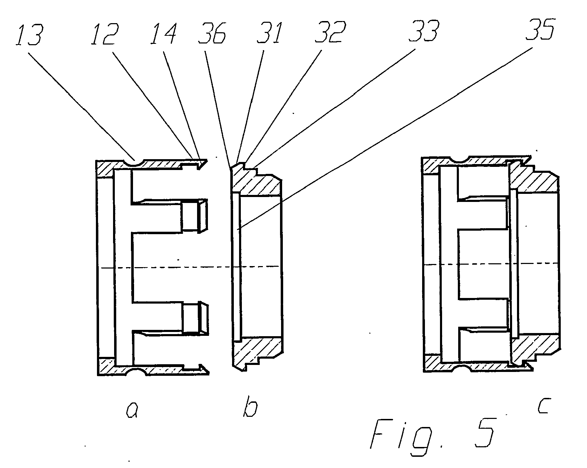

- Fig. 3 b: eine axiale Darstellung eines Kanülenendes mit Kanülenverstärkung ohne Distanzring,
- Fig. 3 c: eine axiale Schnittdarstellung eines Distanzringes,

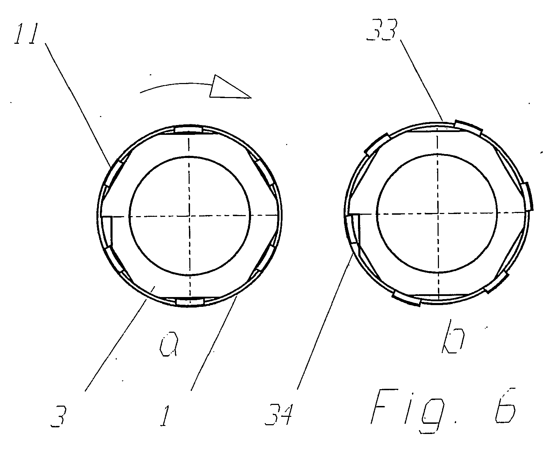

- Fig. 4 a: einen Rastring in perspektivischer Darstellung in Richtung Rohrende gesehen,
- Fig. 4 b: einen Rastring in perspektivischer Darstellung in Richtung Kanülenende gesehen,
- Fig. 5 a: einen axial geschnittenen Klauenring,
- Fig. 5 b: einen axial geschnittenen Rastring,
- Fig. 5 c: einen axial geschnittenen Klauenring und Rastring in eingerastetem Zustand,
- Fig. 6 a: eine Anordnung der Klauen beim Aufschieben auf den Rastring (Raststellung),
- Fig. 6 b: die Anordnung der Klauen auf dem Rastring nach einer Drehbewegung des Klauenringes in Entriegelungsstellung, wobei eine Klaue nicht aufgespreizt ist, an der Drehbegrenzung anliegt und so die Entriegelungsstellung definiert,

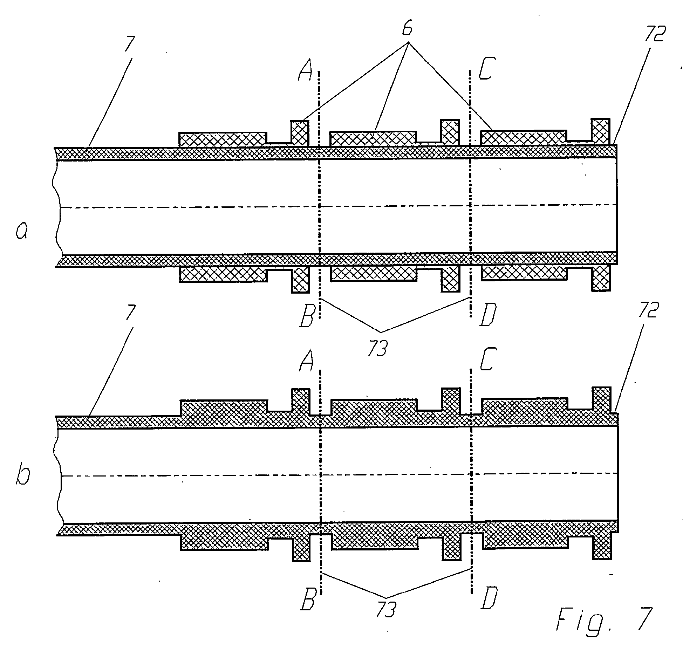

- Fig. 7a: eine Anordnung mehrerer Kanülenverstärkungen auf einer Kanüle,
- Fig. 7b: eine Ausführung einer Kanüle mit durch Spritzguß integral mit der Kanüle verbundenen Verstärkungsformteilen,

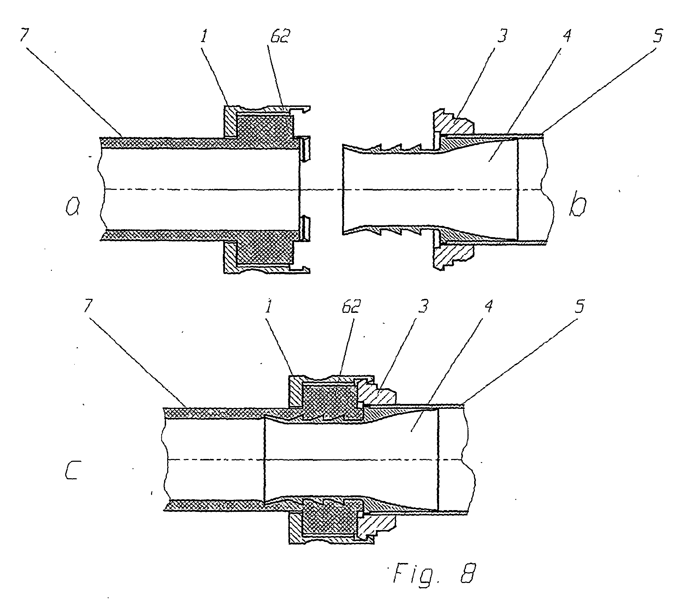

- Fig. 8a: eine Darstellung des Kanülenendes mit Bund und Klauenring,
- Fig. 8b: eine Darstellung des Rohrendes mit Rastring,
- Fig. 8c: eine Darstellung der Verbindung eines Kanülenendes mit einem Rohrende mit einem Anschlag am Kanülenende,

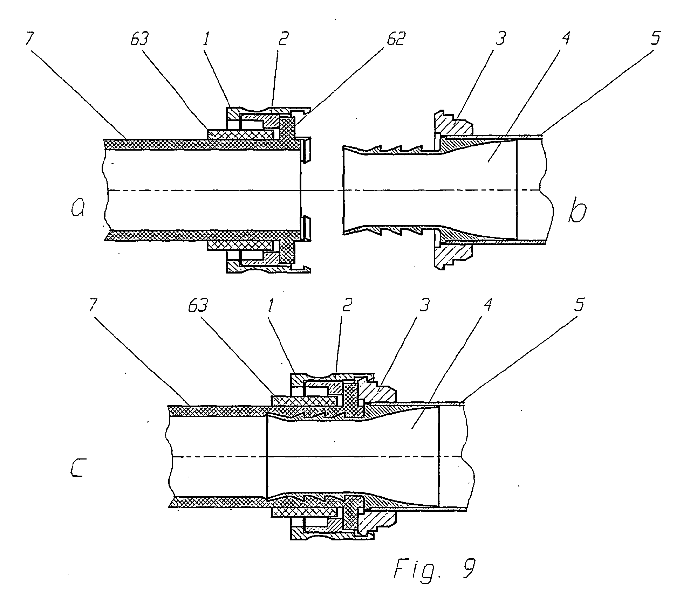

- Fig. 9a: eine Darstellung eines Kanülenendes mit Bund und Spannring sowie Distanzring,
- Fig. 9b: eine Darstellung eines Rohrendes mit Rastring,
- Fig. 9c: eine Darstellung der Verbindung zwischen dem Kanülenende und dem Rohrende mit Bund, Spannring und Distanzring,

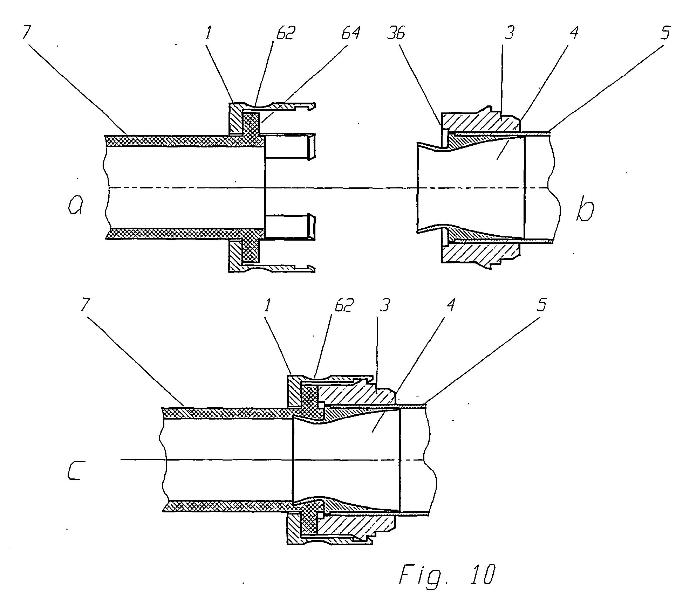

- Fig. 10a: eine Darstellung eines Kanülenendes mit schmalem Bund und Klauenring,
- Fig. 10b: eine Darstellung eines Rohrendes mit verkürztem Rohrstutzen,
- Fig. 10c: eine Anordnung der Verbindung zwischen Kanülenende und Rohrende mit verkürztem Rohrstutzen,

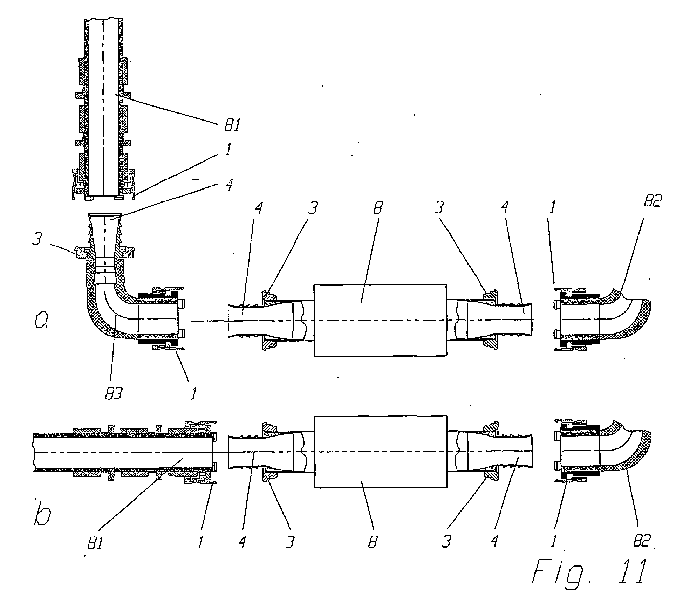

- Fig. 11a: eine schematische Darstellung einer Herzunterstützungspumpe mit den erfindungsgemäßen Verbindungspositionen mit Auslasskrümmer und
- Fig. 11b: eine Herzunterstützungspumpe mit Verbindungspositionen und einer Auslasskanüle mit mehreren Verstärkungsformteilen.

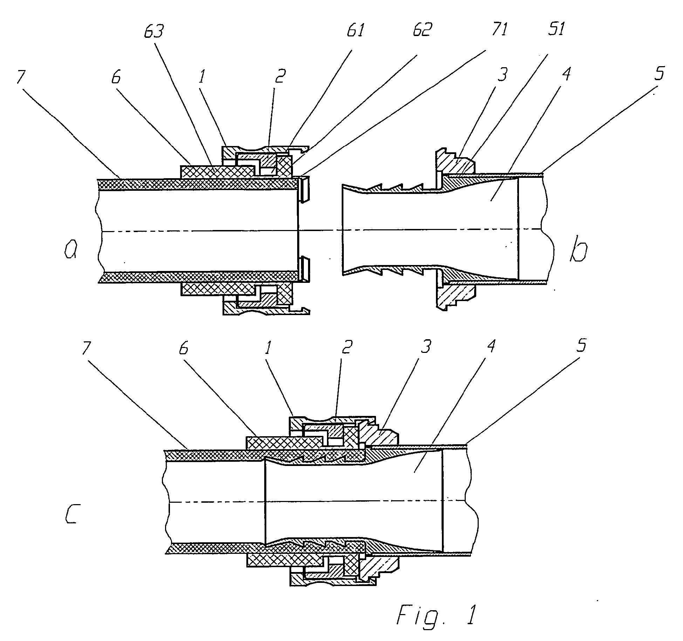

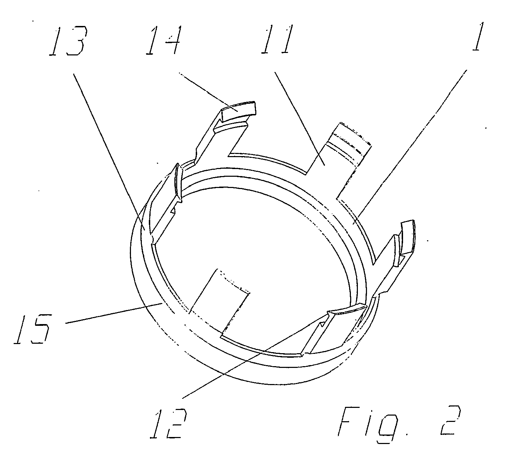

Die Fig. 1a, 1b, 1c zeigen beispielhaft eine erfindungsgemäße Vorrichtung, die eine Verbindung zwischen einer Kanüle 7 und einem Rohr 5 herstellt. Ein Rohrstutzen, hier als Olive 4 ausgebildet, ist in einem Kanülenende 71 eingeschoben (Fig. 1c). Auf dem Kanülenende 71 ist ein Verstärkungsformteil 6 fest mit dem Kanülenende 71 verbunden. In einer Nut 61 des Verstärungsformteils 6 ist ein Distanzring 2 angeordnet. Ein Klauenring 1 ist mit seinen an einem Grundring 15 (vgl. Fig. 2) angebrachten Klauen 11 am Rastring 3 eingerastet. Der Klauenring 1 drückt hierbei auf den Distanzring 2 und dieser auf einen Bund 62 des Verstärkungsformteiles 6, so dass mit einer ringförmigen Stirnfläche 36 des Rastringes 3 eine dichte Verbindung hergestellt wird. Die Dichtung der Kanüle 7 gegenüber der Olive 4 erfolgt durch die elastische Aufweitung des Kanülenendes 71 beim Aufschieben auf die Olive 4, wodurch radiale Dichtkräfte entstehen, die die Innenfläche des Kanülenendes 71 auf die Olive 4 pressen. Die radiale Dichtkraft wird durch den Spannring 63 des Verstärkungsformteils 6 noch erhöht.

Die erfindungsgemäße Vorrichtung ist in ihrer prinzipiellen Funktion eine Schnappverbindung. Die am Grundring 15 angebrachten Klauen 11 werden über den Rastring 3 geschoben und schnappen dort wieder lösbar ein (vgl. Fig. 2, Fig. 5 und Fig. 6).

Die Stirnfläche 36 geht radial nach außen in eine Steigfläche 31 und radial nach innen in eine Freimachung 35 über. Diese dient zur Aufnahme des beim Kürzen der Kanüle während der Implantation eventuell entstehenden Kanülenansatzes 72 vor dem Bund (Fig. 7). Hinter der Stirnfläche 36 befinden sich Halteflächen 32 und Steigflächen 33, in die der auf der Kanüle befindliche Klauenring 1 mit den Klauen 11 einschnappt und so die Kanüle 7 mit dem Rohr 5 verbindet. Der Distanzring 2 und der Klauenring 1 für den Anschluß der Kanüle 7 können sich im Auslieferungszustand schon vormontiert auf der Kanüle 7 befinden oder aber erst während der Operation auf diese montiert werden. Beim Verbinden wird die Kanüle 7 auf die Olive 4 aufgeschoben, wobei sich der Innendurchmesser der Kanüle 7 elastisch weitet. Der Klauenring 1 wird soweit gedreht, dass er relativ zum Rastring 3 in Rastposition steht (Fig. 6a) und dann axial in Richtung auf den Rastring 3 verschoben. Die Klauen 11 werden beim Aufschieben auf die Rastringsteigflächen 31 des Rastringes 3 aufgespreizt und rasten mit Klauenhalteflächen 12 an den Rastringhalteflächen 32 ein (Fig. 5a, 5b, 5c).

In der perspektivischen Darstellung des Klauenringes 1 gem. Fig. 2 sind an einem Grundring 15 sechs Klauen 11 angeordnet. Am Ende der Klauen 11 sind die Klauenhaltefläche 12 und eine Klauensteigfläche 14 vorgesehen. Diese sind für das sichere Einrasten im Rastring 3, wie in Fig. 5a, 5b und 5c dargestellt ist, vorgesehen. Federgelenke 13 des Klauenringes 1 ermöglichen das Auseinanderspreizen der Klauen 11 beim Schnappvorgang auf dem Rastring 3.

In Fig. 3a, 3b und 3c ist das Kanülenende 71 der Kanüle 7 dargestellt. In Fig. 3a ist auf dem Kanülenende 71 das Verstärkungsformteil 6 aufgebracht und in der Nut 61 des Verstärkungsformteiles 6 sitzt der Distanzring 2. Fig. 3b zeigt das Kanülenende 71 ohne Distanzring 2 aber mit Verstärkungsformteil 6 mit den ausgebildeten Spannring 63, Bund 62 und Nut 61. Fig. 3c zeigt den Distanzring 2, der formschlüssig in die Nut 61 des Verstärkungsformteiles 6 eingebracht werden kann.

In Fig. 4a und 4b ist ein einzelner Rastring 3 gezeigt, an dem Rastringsteigflächen 31, Rastringhaltefläche 32, Entriegelungssteigflächen 33, eine Drehbegrenzung 34, eine Freimachung 35 und eine Stirnfläche 36, die als axiale Dichtfläche dienen kann, dargestellt sind. Fig. 4a zeigt die Darstellung in Richtung Rohr 5 und Fig. 4b die Darstellung in Richtung Kanüle 7 gesehen.

In Fig. 5a, 5b und 5c ist ein Klauenring 1 und ein Rastring 3 einmal im nichteingerasteten bzw. nichteingeschnappten Zustand (Fig. 5a und 5b) und einmal im eingerasteten bzw. eingeschnappten Zustand (Fig. 5c) dargestellt. Hier sind noch einmal das Zusammenwirken des Federgelenkes 13 der Klauenhaltefläche 12 sowie der Klauensteigfläche 14 des Klauenringes 1 mit der Steigfläche 31, der Haltefläche 32, der Steigfläche für Entriegelung 33 sowie der Freimachung 35 erkennbar.

In Fig. 6a ist der eingerastete Zustand (Rastzustand) nach dem Aufschieben des Klauenrings 1 auf den Rastring 3 dargestellt. Durch Drehung des Klauenrings 1 bis zu einer Drehbegrenzung 34 wird die Entriegelungsposition erreicht, wobei die Klauen 11 durch Gleiten auf den Entriegelungssteigflächen 33 des Rastringes 3 aufgespreizt werden (Fig. 6b). Der Klauenring 1 kann nun axial vom Rastring 3 und die Kanüle 7 von der Olive abgezogen und so die Verbindung gelöst werden.

In Fig. 7a und 7b ist die Anordnung mehrerer Verstärungsformteile 6 auf der Kanüle 7 zur vorteilhaften Anpassung der Länge beim Implantieren dargestellt. Je nach anatomischen Gegebenheiten wird die Länge der Kanüle 7 gekürzt, indem eine bestimmte Anzahl der Verstärkungsformteile 6 während der Operation abgeschnitten wird. Der ggf. dabei entstehende Kanülenansatz 72 ragt beim Fügen von Kanüle 7 und Rohr 5 in die Freimachung 35 des Rastringes 3. Fig. 7b zeigt eine Kanüle, deren Verstärkungsformteile 6 integraler Bestandteil der Kanüle sind (Spritzgußherstellung).

Die möglichen Schnittstellen beim Anpassen an die Größenverhältnisse bei der Implantation sind durch A-B und C-D gekennzeichnet.

In Fig. 8a, 8b und 8c ist eine weitere Variante der erfindungsgemäßen Vorrichtung dargestellt. Fig. 8a zeigt das Kanülenende 71 der Kanüle 7 mit Rastring 1 und dem Bund 62, der hier unmittelbar an das Kanülenende 71 angeformt ist. Bei dieser Variante ist ein Verstärkungsformteil 6 nicht vorgesehen.

In Fig. 8b ist das Rohrende 51 des Rohres 5 dargestellt, das den Rastring 3 und die Olive 4 aufweist. Durch Eischieben der Olive 4 in das Kanülenende 71 wird durch die Einrastung des Klauenringes 1 am Rastring 3 eine dichte Verbindung zwischen beiden hergestellt. Der Bund 62 ist hier relativ breit angelegt, so dass gleichzeitig eine Verstärkung der radialen Dichtkräfte in Verbindung mit der axialen Abdichtung an den Berührungsflächen der Enden erfolgt.

In Fig. 9a, 9b und 9c ist eine weitere Ausgestaltung der erfindungsgemäßen Vorrichtung dargestellt. Anstelle eines geschlossenen Verstärkungsformteiles 6 ist hier die Anordnung eines getrennten Bundes 62, Spannringes 63 und Distanzringes 2 vorgesehen. Auch mit dieser Variante wird eine feste Verbindung und Abdichtung erreicht.

In Fig. 10a, 10b und 10c ist eine weitere Ausgestaltung der erfindungsgemäßen Vorrichtung dargestellt. Hierbei ist die Olive 4 deutlich verkürzt, während der Rastring 3 eine breitere Form aufweist.

In Fig. 11a und 11b ist schematisch der Einsatz der erfindungsgemäßen Vorrichtung an einer implantierbaren Blutpumpe dargestellt.

So zeigt Fig. 11a eine Blutpumpe 8, die an ihren Aus- und Eingängen die Oliven 4 aufweist. Zum Auslasskrümmer 83 ist mittels der erfindungsgemäßen Vorrichtung durch Einrastung des Klauenringes 1 am Rastring 3 eine feste Verbindung herstellbar. Auf der anderen Seite der Pumpe 8 ist ebenfalls durch Einrastung des Klauenringes 1 am Rastring 3 eine Einlasskanüle mit der Pumpe verbindbar. Darüber hinaus ist es möglich, den Auslasskrümmer an seinem anderen Ende mit einer sogenannten Auslasskanüle 81 zu verbinden, hier an der Darstellung gemäß Fig. 11a ist insbesondere der große Vorteil der Vorrichtung zu erkennen. Durch Verdrehung der Krümmer ist die optimale Position zu erreichen, ohne dass die Verbindbarkeit mit der Pumpe 8 in irgendeiner Weise beeinträchtigt wird.

In Fig. 11b ist die Verbindung der Pumpe 8 mit den Oliven 4 mit der Auslasskanüle 81 und der Einlasskanüle 82 ohne Auslasskrümmer 83 dargestellt. Die Auslasskanüle 81 weist mehrere am Kanülenende 71 hintereinander angeordnete Verstärkungsformteile 6 auf (Fig.7a und 7b).

Die Anwendung erfolgt vorteilhafterweise insbesondere bei einer Blutpumpe 8, die im Körper implantiert werden und die mit Einlass- und Auslasskanülen 82 und 81 verbunden werden müssen. Sowohl Einlass- als auch Auslasskanüle 82 und 81 werden über den erfindungsgemäßen Schnappverbinder mit dem Pumpensystem verbunden. Das vom Herz kommende Blut wird über die Einlasskanüle 82 der Blutpumpe 8 zugeführt und von der Blutpumpe 8 über die Auslasskanüle 81 der hier nicht dargestellten Aorta zugeleitet. Einlass- und Auslasskanüle 82 und 81 sind über die erfindungsgemäße Vorrichtung mit der Blutpumpe 8 verbunden. Zwischen Blutpumpe 8 und Auslasskanüle 81 kann wahlweise der Auslasskrümmer 83 eingefügt werden. Dessen Eingang ist wie ein Kanülenende 71 ausgebildet und kann so ebenfalls mit einem Schnappverbinder am Pumpenausgang befestigt werden. Der Ausgang des Auslasskrümmers 83 weist die Olive 4 auf und kann somit mit einem weiteren Schnappverbinder mit der Auslasskanüle 81 verbunden werden (Fig. 11a).

Bei der Implantation des Herzunterstützungssystemes ist es möglich, durch Abschneiden mindestens eines Verstärkungsformteiles 6, wie in Fig. 7a gezeigt, eine Anpassung des Systems an die jeweiligen körperlichen Größenverhältnisse zu erreichen. Der Klauenring 1 und der Distanzring 2 (Fig. 1) können auf das danach folgende Verstärkungsformteil 6 durch entsprechendes Verformen des elastischen Materials ohne weiteres aufgeschoben werden.

### Bezugszeichenliste

- 1: Klauenring
- 11: Klaue
- 12: Klauenhaltefläche
- 13: Federgelenk
- 14: Klauensteigfläche
- 15: Grundring

- 2: Distanzring

- 3: Rastring
- 31: Rastringsteigfläche
- 32: Rastringhaltefläche
- 33: Entriegelungssteigflächen
- 34: Drehbegrenzung
- 35: Freimachung
- 36: Stirnfläche

- 4: Olive

- 5: Rohr
- 51: Rohrende

- 6: Verstärkungsformteil
- 61: Nut
- 62: Bund
- 63: Spannring
- 64: Bundstirnfläche

- 7: Kanüle
- 71: Kanülenende
- 72: Kanülenansatz
- 73: Trennstelle

- 8: Blutpumpe
- 81: Auslasskanüle
- 82: Einlasskanüle
- 83: Auslasskrümmer

## Patentansprüche

1. Vorrichtung zum Verbinden von einer aus flexiblem Material bestehenden Kanüle mit einem Rohr, wobei ein am Rohr (5) angesetzter Rohrstutzen (4) in die Kanüle (7) eingeschoben ist,
**dadurch gekennzeichnet, dass**
ein auf einem Kanülenende (71) bis zu einem Anschlag (2, 62) verschiebbar und drehbar angeordneter Klauenring (1) mit einem auf einem Rohrende (51) befestigten Rastring (3) durch axiales Verschieben und Einrastung am Rastring (3) verbindbar ist und die Kanüle (7) an ihrem Kanülenende (71) einen Spannring (63) aufweist, auf dem der Klauenring (1) verschiebbar und drehbar angeordnet ist.

2. Vorrichtung nach Anspruch 1, **dadurch gekennzeichnet, dass** der Spannring (63) aus elastischem Material, z.B. Silikon, besteht.

3. Vorrichtung nach Anspruch 1, **dadurch gekennzeichnet, dass** der Spannring (63) aus unflexiblem Material, z.B. Metall, besteht.

4. Vorrichtung nach einem der Ansprüche 1 bis 3, **dadurch gekennzeichnet, dass** der Spannring (63) als geschlitzte Hülse ausgeführt ist.

5. Vorrichtung nach einem der Ansprüche 1 bis 4, **dadurch gekennzeichnet, dass** der Anschlag als endständiger Bund (62) am Kanülenende (71) ausgebildet ist.

6. Vorrichtung nach einem der Ansprüche 1 bis 5, **dadurch gekennzeichnet, dass** vor dem Bund (62) ein Distanzring (2) dreh- und verschiebbar angeordnet ist.

7. Vorrichtung nach Anspruch 1, **dadurch gekennzeichnet, dass** auf dem Kanülenende (71) ein Verstärkungsformteil (6) angeordnet ist, das mit der Kanüle (7) fest verbunden ist und auf dem der Klauenring (1) verschiebbar und drehbar angeordnet ist.

8. Vorrichtung nach Anspruch 1 oder 7, **dadurch gekennzeichnet, dass** das Verstärkungsformteil (6) einen Spannring (63) und einen Bund (62) aufweist.

9. Vorrichtung nach einem der Ansprüche 1, 7 oder 8, **dadurch gekennzeichnet, dass** das Verstärkungsformteil (6) zwischen Spannring (63) und Bund (62) eine Nut (61) aufweist.

10. Vorrichtung nach einem der Ansprüche 1, 7 bis 9, **dadurch gekennzeichnet, dass** in der Nut (61) des Verstärkungsformteiles (6) der Distanzring (2) angeordnet ist.

11. Vorrichtung nach einem der Ansprüche 1 bis 10, **dadurch gekennzeichnet, dass** der Klauenring (1) einen Grundring (15) aufweist, von dem ausgehend, mindestens zwei Klauen (11) mit Klauenhalteflächen (12) über Federgelenke (13) am Grundring (15) angeordnet sind.

12. Vorrichtung nach Anspruch 11, **dadurch gekennzeichnet, dass** der Distanzring (2) fest mit dem Grundring (15) verbunden ist.

13. Vorrichtung nach einem der Ansprüche 1 bis 12, **dadurch gekennzeichnet, dass** der auf dem Rohrende (51) fixierte Rastring (3) Rastring-Steigflächen (31) und Rastring-Halteflächen (32) aufweist.

14. Vorrichtung nach einem der Ansprüche 1, 8 bis 13, **dadurch gekennzeichnet, dass** der Klauenring (1) auf dem Spannring (63) des Verstärkungsformteiles (6) bis zum Anschlag am Distanzring (2) und/oder Bund (62) axial verschiebbar und drehbar angeordnet ist.

15. Vorrichtung nach einem der Ansprüche 1, 8 bis 14, **dadurch gekennzeichnet, dass** das Verstärkungsformteil (6) aus flexiblem Material besteht.

16. Vorrichtung nach einem der Ansprüche 1, 8 bis 15, **dadurch gekennzeichnet, dass** das Verstärkungsformteil (6) und die Kanüle (7) aus dem gleichen flexiblen Material bestehen.

17. Vorrichtung nach Anspruch 1 bis 16, **dadurch gekennzeichnet, dass** der Rastring (3) Entriegelungssteigflächen (33) aufweist.

18. Vorrichtung nach Anspruch 1 bis 17, **dadurch gekennzeichnet, dass** der Rastring (3) mindestens eine Drehbegrenzung (34) aufweist.

19. Vorrichtung nach Anspruch 1 bis 18, **dadurch gekennzeichnet, dass** der Rastring (3) eine Freimachung (35) aufweist.

## Claims

1. Device for connection of a cannula made from a flexible material to a tube, whereby a nipple (4) located on the tube (5) is pushed into the cannula (7), **characterised in that** a claw ring (1) rotatably and displaceably positioned on one cannula end (71) up to a stop (2, 62) can be connected with a locking ring (3) fitted to a tube end (51) by means of axial displacement and arrested on the locking ring (3), and the cannula (7) comprises a tension ring (63) on its cannula end (71), on which the claw ring (1) is displaceably and rotatably connected.

2. Device according to Claim 1, **characterised in that** the tension ring (63) consists of an elastic material, for example silicon.

3. Device according to Claim 1, **characterised in that** the tension ring (63) consists of a non-flexible material, for example metal.

4. Device according to one of the Claims 1 to 3, **characterised in that** the tension ring (63) takes the form of a slotted sleeve.

5. Device according to one of the Claims 1 to 4, **characterised in that** the stop takes the form of an end sided collar (62) on the cannula end (71).

6. Device according to one of the Claims 1 to 5, **characterised in that** a spacer ring (2) is rotatably and displaceably positioned in front of the collar (62).

7. Device according to Claim 1, **characterised in that** a formed reinforcement element (6) is located on the cannula end (71), the same being firmly connected to the cannula (7) and displaceably and rotatably positioned on the claw ring (1).

8. Device according to Claim 1 or 7, **characterised in that** the formed reinforcement element (6) comprises a tension ring (63) and a collar (62).

9. Device according to one of the Claims 1, 7 or 8, **characterised in that** the formed reinforcement element (6) comprises a groove (61) between the tension ring (63) and the collar (62).

10. Device according to one of the Claims 1, 7 to 9, **characterised in that** the spacer ring (2) is located in the groove (61) of the formed reinforcement element (6).

11. Device according to one of the Claims 1 to 10, **characterised in that** the claw ring (1) comprises a base ring (15), from which at least two claws (11) extend with claw holding surfaces (12) via resilient joints (13) on the base ring (15).

12. Device according to Claim 11, **characterised in that** the spacer ring (2) is firmly connected to the base ring (15).

13. Device according to one of the Claims 1 to 12, **characterised in that** the locking ring (3) fitted on the tube end (51) comprises locking ring increase steps (31) and locking ring holding surfaces (32).

14. Device according to one of the Claims 1, 8 to 13, **characterised in that** the claw ring (1) is located axially displaceable and rotatable on the tension ring (63) of the formed reinforcement element (6) up to the stop of the spacer ring (2) and/or the collar (62).

15. Device according to one of the Claims 1, 8 to 14, **characterised in that** the formed reinforcement element (6) consists of a flexible material.

16. Device according to one of the Claims 1, 8 to 15, **characterised in that** the formed reinforcement element (6) and the cannula (7) consist of the same flexible material.

17. Device according to Claim 1 to 16, **characterised in that** the locking ring (3) comprises sloped unlatching faces (33).

18. Device according to Claim 1 to 17, **characterised in that** the locking ring (3) comprises at least one rotation limit stop (34).

19. Device according to Claim 1 to 18, **characterised in that** the locking ring (3) comprises a clearance (35).

## Revendications

1. Dispositif permettant de relier une canule constituée d'un matériau souple à un tube, un embout (4) fixé sur le tube (5) étant inséré dans la canule (7), **caractérisé en ce qu'**une bague à griffes (1) disposée de manière à pouvoir tourner et à pouvoir se déplacer sur une extrémité de la canule (71) jusqu'à une butée (2, 62) est à même d'être reliée à une bague d'arrêt (3) fixée à une extrémité du tube (51) grâce à un déplacement axial et à un encliquetage sur la bague d'arrêt (3), et la canule présente une bague de serrage (63) à son extrémité de canule (71), bague de serrage sur laquelle est disposée la bague à griffes (1) en pouvant se déplacer et en pouvant tourner.

2. Dispositif selon la revendication 1, **caractérisé en ce que** la bague de serrage (63) est constituée d'un matériau élastique, par exemple de la silicone.

3. Dispositif selon la revendication 1, **caractérisé en ce que** la bague de serrage (63) est constituée d'un matériau non souple, par exemple un métal.

4. Dispositif selon l'une quelconque des revendications 1 à 3, **caractérisé en ce que** la bague de serrage (63) est configurée sous la forme d'une douille fendue.

5. Dispositif selon l'une quelconque des revendications 1 à 4, **caractérisé en ce que** la butée est configurée sous la forme d'un collet terminal (62) à l'extrémité de la canule (71).

6. Dispositif selon l'une quelconque des revendications 1 à 5, **caractérisé en ce que**, avant le collet (62), une bague d'écartement (2) est disposée de manière à pouvoir se déplacer et à pouvoir tourner.

7. Dispositif selon la revendication 1, **caractérisé en ce qu'**à l'extrémité de la canule (71) est disposée une pièce moulée de renforcement (6), qui est reliée fermement à la canule (7) et est disposée de manière à pouvoir se déplacer et à pouvoir tourner sur la bague à griffes (1).

8. Dispositif selon la revendication 1 ou 7, **caractérisé en ce que** la pièce moulée de renforcement (6) présente une bague de serrage (63) et un collet (62).

9. Dispositif selon l'une quelconque des revendications 1, 7 ou 8, **caractérisé en ce que** la pièce moulée de renforcement (6) présente une rainure (61) entre la bague de serrage (63) et le collet (62).

10. Dispositif selon l'une quelconque des revendications 1, 7 à 9, **caractérisé en ce que**, dans la rainure (61) de la pièce moulée de renforcement (6), est disposée la bague d'écartement (2).

11. Dispositif selon l'une quelconque des revendications 1 à 10, **caractérisé en ce que** la bague à griffes (1) présente une bague de fond (15), et au moins deux griffes (11), présentant des surfaces de maintien de griffe (12), sont disposées sur la bague de fond (15) en partant de celle-ci par l'intermédiaire d'articulations élastiques (13).

12. Dispositif selon la revendication 11, **caractérisé en ce que** la bague d'écartement (2) est reliée fermement à la bague de fond (15).

13. Dispositif selon l'une quelconque des revendications 1 à 12, **caractérisé en ce que** la bague d'arrêt (3) fixée à l'extrémité du tube (51) présente des surfaces ascendantes de bague d'arrêt (31) et des surfaces de maintien de bague d'arrêt (32).

14. Dispositif selon l'une quelconque des revendications 1, 8 à 13, **caractérisé en ce que** la bague à griffes (1) est disposée de manière à pouvoir tourner et se déplacer axialement sur la bague de serrage (63) de la pièce moulée de renforcement (6) jusqu'à la butée sur la bague d'écartement (2) et/ou collet (62).

15. Dispositif selon l'une quelconque des revendications 1, 8 à 14, **caractérisé en ce que** la pièce moulée de renforcement (6) est constituée d'un matériau souple.

16. Dispositif selon l'une quelconque des revendications 1, 8 à 15, **caractérisé en ce que** la pièce moulée de renforcement (6) et la canule (7) sont constituées du même matériau souple.

17. Dispositif selon l'une quelconque des revendications 1 à 16, **caractérisé en ce que** la bague d'arrêt (3) présente des surfaces ascendantes de déverrouillage (33).

18. Dispositif selon l'une quelconque des revendications 1 à 17, **caractérisé en ce que** la bague d'arrêt (3) présente au moins un limiteur de rotation (34).

19. Dispositif selon l'une quelconque des revendications 1 à 18, **caractérisé en ce que** la bague d'arrêt (3) présente un dégagement (35).
